(19) **Europäisches Patentamt / European Patent Office / Office européen des brevets**

(11) **EP 4 111 461 B1**

(12) **EUROPEAN PATENT SPECIFICATION**

(45) Date of publication and mention
of the grant of the patent:
**03.12.2025 Bulletin 2025/49**

(21) Application number: **20781563.0**

(22) Date of filing: **06.10.2020**

(51) International Patent Classification (IPC):
**G16H 20/40** *(2018.01)*    **G16H 40/63** *(2018.01)*

(52) Cooperative Patent Classification (CPC):
**G16H 20/40; G16H 40/63**

(86) International application number:
**PCT/EP2020/077935**

(87) International publication number:
**WO 2021/170257 (02.09.2021 Gazette 2021/35)**

(54) **METHOD FOR CALCULATING THE PROXIMAL AND DISTAL ENDS OF AN INTERLACED DEVICE BEFORE BEING POSITIONED IN A VASCULAR STRUCTURE AND COMPUTER PROGRAMS THEREOF**

VERFAHREN ZUR BERECHNUNG DER ANFANGS- UND ENDPOSITIONEN EINER VERSCHACHTELTEN VORRICHTUNG VOR DER POSITIONIERUNG IN EINER GEFÄSSSTRUKTUR UND COMPUTERPROGRAMME DAFÜR

PROCÉDÉ DE CALCUL DES POSITIONS INITIALES ET FINALES D'UN DISPOSITIF ENTRELACÉ AVANT D'ÊTRE POSITIONNÉ DANS UNE STRUCTURE VASCULAIRE ET PROGRAMMES INFORMATIQUES ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2020 EP 20382143**

(43) Date of publication of application:
**04.01.2023 Bulletin 2023/01**

(73) Proprietor: **Mentice AB**
**411 03 Gothenburg (SE)**

(72) Inventors:
• **MUÑOZ, Romina Luciana**
**Buenos Aires, 7000 (AR)**
• **LARRABIDE, Ignacio**
**Buenos Aires, 7000 (AR)**
• **FERNÁNDEZ MARTÍNEZ, Héctor**
**08026 Barcelona (ES)**

(74) Representative: **Torner, Juncosa I Associats, SL**
**C / Pau Claris, 108, 1r 1a**
**08009 Barcelona (ES)**

(56) References cited:
**US-A1- 2014 350 350    US-A1- 2016 232 659**
**US-A1- 2017 095 254    US-A1- 2019 038 358**

**Description**

Technical Field

The present invention relates to a computational method and computer products for calculating the proximal and distal ends of a device of interlaced threads (or interlaced device) with one or more sections with a singularity, before being positioned in a vascular structure. The invention allows knowing the configuration the interlaced device will adopt before it is implanted. The invention also allows calculating the local porosity of the device after it is implanted/positioned.

By singularity it should be understood a point, or section, along the longitudinal direction of the interlaced device, where multiple (at least three) of the interlaced filaments/threads coincide. That is, because of the singularity/singularities the interlaced device is "closed" at some point where (all or at least most of) the threads converge. The device can be closed at one of its ends, at both ends, or in more sites.

Background of the Invention

Examples of interlaced devices are those used in the treatment of vascular pathologies when deployed inside a vessel. More particularly, examples of devices of this type are the intrasaccular devices described in patent application US 20120283768-A1 and in patent US 10136896-B2.

Likewise, a tool which allows a user to manually position a device with singularities inside vascular models, but without simulating the deployment method is disclosed in papers [1] and [2]. This tool is based on deformation by means of a spring model.

Furthermore, patent US 10176566-B2 proposes a method for determining the final length of a stent before the positioning thereof in a vascular structure. The present invention extends and adapts what is described in this US patent.

Likewise, the US 2019038358-A1 provides a method of estimating the length of a stent. Unlike present invention, the stent of this US patent application does not have any singularity in the end sections thereof.

Therefore, new methods which allow calculating the final positions of interlaced devices with one or more singularities are required.

References:

[1] JR Cebral, et al. Analysis of flow dynamics and outcomes of cerebral aneurysms treated with intrasaccular flow-diverting devices. American Journal of Neuroradiology, 2019.

[2] Fernando Mut, et al. Image-based modeling of blood flow in cerebral aneurysms treated with intrasaccular flow diverting devices. International journal for numerical methods in biomedical engineering, page e3202, 2019.

Description of the Invention

To that end, embodiments of the present invention provide according to a first aspect a method for calculating the proximal and distal ends (i.e. the final positions) of an interlaced device before being positioned in a vascular structure. The method comprises performing the following steps by a computer including one or more processors and at least one memory:

- receiving a three-dimensional image of a vascular structure in which the device formed by interlaced threads (hereinafter interlaced device) will be positioned, and tracing a central line of said vascular structure in the three-dimensional image which defines a direction in which the interlaced device is to be deployed, wherein the interlaced device includes at a proximal end or at a distal end thereof a section with a singularity (i.e. a zone where a plurality of the interlaced threads coincide);
- defining, based on an input provided by a user, a distal point $P_d$ on the traced central line and a local morphology of the vessel (i.e. the shape of the vessel in the vicinity of a given point), wherein the distal point $P_d$ indicates the point where the distal end of the interlaced device will start to be deployed; and
- calculating a proximal point $P_p$ using the distal point $P_d$ and the local morphology of the vessel, both having been defined, wherein the proximal point $P_p$ indicates the point that limits a portion of the central line over the traced central line that will be needed for deploying the section of the interlaced device including the singularity.

If the singularity of the interlaced device is at the distal end, the method further comprises extracting a morphological descriptor $m_d$ of the vascular structure at the distal point $P_d$ and comparing said morphological descriptor $m_d$ with a nominal morphological descriptor $M_n$ of a distal section of the interlaced device including the singularity. Then, a point $P_a$ is defined as $P_a = P_d$.

In the event that the morphological descriptor $m_d$ is smaller than the nominal morphological descriptor $M_n$, the method further comprises:

i. making the point $P_a$ equal to a point next to $P_a$ in a proximal direction along the traced central line,
ii. calculating a local morphological descriptor $m_a$ of a cross-section of the vascular structure at said point $P_a$,
iii. calculating a distance $h_a$ as $h_a = M_n * dumping(m_a)$, where $dumping(m)$ is a mathematical function in the interval [0,1] which considers the variation of $h_a$ according to the expansion of said distal section of the interlaced device including the singularity to an expansion diameter corresponding to the morphological descriptor $m_a$,
iv. identifying a point $P_{am}$ that is located an interval (or length) $h_a$ from the distal point $P_d$ and on a plane perpendicularly intersecting the traced central line at point $P_a$,
v. calculating $d_d$ as the distance between the point $P_a$ and the point $P_{am}$, and
vi. comparing the calculated distance $d_d$ with the local morphological descriptor $m_a$, wherein:
if the distance $d_d$ is smaller than the local morphological descriptor $m_a$, steps i. to v. are repeated, and if the distance $d_d$ is greater than or equal to the local morphological descriptor $m_a$, $P_p = P_a$ is defined as the proximal point that limits a portion of the central line over the traced central line that will be needed for deploying the distal section of the interlaced device including the singularity.

In the event that the morphological descriptor $m_d$ is greater than or equal to the nominal morphological descriptor $M_n$, the method comprises selecting the proximal point $P_p$ as the point that is located a distance $d_{min}$ from the distal point $P_d$ in the proximal direction, where $d_{min}$ is the minimum height, over the traced central line, achieved by said distal section, corresponding to the height of the distal section being in the configuration corresponding to the nominal morphological descriptor $M_n$ (i.e. the nominal configuration, where the interlaced device is open to its maximum diameter, without constraints. Indeed, $d_{min}$ is a constant determined by the design of the device).

In contrast, if the singularity is at the proximal end, the method further comprises:

- extracting a morphological descriptor $m_d$ of the vascular structure at the distal point $P_d$;
- calculating a distance $h_d$ as $h_d = M_n * dumping(m_d)$, where $dumping(m)$ is a mathematical function in the interval [0,1] which considers the variation of $h_d$ according to the expansion of a proximal section of the interlaced device including the singularity to an expansion diameter corresponding to the morphological descriptor $m_d$; and
- comparing said morphological descriptor $m_d$ with a nominal morphological descriptor $M_n$ of the proximal section of the interlaced device including the singularity.

Then, a point $P_a$ is defined as $P_a = P_d$, and a point $P_{dm}$ that is located an interval $m_d$ from the distal point $P_d$ and on a plane perpendicularly intersecting the traced central line at the distal point $P_d$ is identified.

In the event that the morphological descriptor $m_d$ is smaller than the nominal morphological descriptor $M_n$, the method further comprises:

vii. making the point $P_a$ equal to a point next to $P_a$ in a proximal direction along the traced central line,
viii. calculating $d_a$ as the distance between the point $P_a$ and the point $P_{dm}$, and
ix. comparing the calculated distance $d_a$ with the distance $h_d$, wherein:
if the distance $d_a$ is smaller than the $h_d$, steps vii. and viii. are repeated, and if the distance $d_a$ is greater than or equal to the distance $h_d$, $P_p = P_a$ is defined as the proximal point that limits a portion of the central line over the traced central line that will be needed for deploying the proximal section of the interlaced device including the singularity.

In the event that the morphological descriptor $m_d$ is greater than or equal to the nominal morphological descriptor $M_n$, the method comprises selecting the proximal point $P_p$ as the point that is located a distance $d_{min}$ from the distal point $P_d$ in the proximal direction, where $d_{min}$ is the minimum height, over the traced central line, achieved by said proximal section, corresponding to the height of said proximal section being in the configuration corresponding to the morphological descriptor $m_d$.

In one embodiment, the interlaced device has a singularity in the proximal section and a singularity in the distal section, therefore the calculation of the proximal and distal ends of the interlaced device is performed for both proximal and distal sections, according to the steps described above. In this case, however, the distal point $P_d$ of the proximal section is defined as the proximal point $P_p$ of the distal section.

In one embodiment, the interlaced device further has a (tubular-shaped) central section. The traced central line of the vascular structure is also divided into different segments. The method in this case further comprises:

x. selecting a point $P_c$ of the central line at which the deployment of the device of the distal section ended, wherein the point $P_c$ is the proximal point $P_p$ of the distal section;

xi. extracting from the central line at least one morphological descriptor $m_c$ of the segment corresponding to point $P_c$;

xii. calculating a height of the interlaced device for a first segment using a ratio indicating a change in height of the interlaced device according to the local morphology of the vascular structure;

xiii. subtracting said calculated height from a nominal height of the interlaced device, obtaining a new nominal height,

wherein if said new nominal height is greater than 0, steps xi) to xiii) are repeated for the segment contiguous to the preceding segment, moving forward in the proximal direction, and if the new nominal height is approximately 0, all the lengths of each segment are added together, this sum being the final height of the interlaced device after its positioning.

In one embodiment, the interlaced device is attached to a second interlaced device (of the same diameter) at either the proximal end or distal end thereof. The second interlaced device can include a singularity in its proximal section, in its distal section, or in both. In this case, the proposed method comprises performing the calculation of the proximal and distal ends of the second interlaced device according to the steps described above, depending on where the singularity/singularities is/are located.

According to the proposed method, the morphological descriptors $m_d$ and $m_a$ can include: the minimum radius of the cross-section of the vasculature perpendicular to the central line, or the maximum radius, or the radius of the equivalent circumference with the perimeter equal to the cross-section perpendicular to the central line, or the radius of the equivalent circumference with an area equal to the cross-section perpendicular to the central line, among others.

In one embodiment, the method further comprises calculating the porosity on the entire surface of the interlaced device. To that end, the method comprises:

- dividing the surface of the singularity into a specific number of portions, wherein said number of portions have a common center at the distal point $P_d$, cover the entirety of said surface, and depend on the number of interlaced threads constituting the interlaced device;
- dividing the surface of the singularity into concentric circumferences, wherein said circumferences have a common center at the distal point $P_d$;
- dividing the surface into a plurality of cells, wherein each cell is obtained considering the section of one of said portions contained between two consecutive concentric circumferences;
- calculating for each of the plurality of cells: the total area, the area of the thread going through the cell and the uncovered area; and
- calculating the porosity of each cell as the ratio between the uncovered area divided by the total area of the cell or the ratio between the covered area divided by the total area of the cell.

In the previous embodiment, all the portions can have the same or a different size. In particular, the number of portions is equal to the number of threads.

Other embodiments of the invention disclosed herein also include computer program products for performing the steps and operations of the proposed method in the first aspect of the invention. More particularly, a computer program product is an embodiment having a computer-readable medium including computer program instructions encoded therein which, when executed in at least one processor of a computer system, cause the processor to perform the operations indicated herein as embodiments of the invention.

The present invention thus provides a fast method to simulate intra-sacular devices inside the patient anatomy that allows knowing, in a quick and precise way, how a specific device size adapts to the aneurysm morphology. That is, the present invention allows knowing, before being implanted, the final arrangement of a device with singularities when it is positioned inside an aneurysm. This involves, in addition to the possibility of evaluating the apposition with respect to the walls of the aneurysm achieved by the device, the location of the proximal end thereof inside the aneurysm. This location is important because it helps the neurointerventionalist to make decisions about the device that is to be used. The device is ideally expected to completely and safely cover the neck of the aneurysm to interrupt the blood flow inside the aneurysm.

Brief Description of the Drawings

The foregoing and other features and advantages will be better understood from the following merely illustrative and non-limiting detailed description of several embodiments in reference to the attached drawings, in which:

Fig. 1 schematically illustrates an interlaced device divided into three sections, according to an embodiment of the present invention.

Fig. 2 is a flowchart illustrating a method for calculating the proximal and distal ends of an interlaced device before being positioned in a vascular structure, according to an embodiment of the present invention.

Fig. 3 is a flowchart illustrating a method for calculating the proximal and distal ends of an interlaced device before being positioned in a vascular structure when the interlaced device includes a singularity at its distal end, according to an embodiment of the present invention.

Fig. 4 is a flowchart illustrating a method for calculating the proximal and distal ends of an interlaced device before being positioned in a vascular structure when the interlaced device includes a singularity at its proximal end, according to an embodiment of the present invention.

Fig. 5 is a flowchart illustrating the calculation for the central section of the interlaced device, according to an embodiment of the present invention.

Fig. 6 illustrates the step-by-step virtual deployment of the interlaced device inside a vascular structure. Fig. 6A shows the extraction of the central line; Fig. 6B shows the deployment of the distal section with a singularity; Fig. 6C shows the deployment of the central section next to the previously deployed section; Fig. 6D shows the deployment of the proximal section with a singularity next to the previously deployed sections, imparting the final configuration of the device.

Fig. 7 is a flowchart illustrating a method for calculating the porosity of an interlaced device with one or more singularities, according to an embodiment of the present invention.

Figs. 8A-8C graphically show some of the steps implemented by the proposed method for calculating the porosity of an interlaced device with one or more singularities, according to an embodiment of the present invention.

Figs. 9A-9C illustrate an example of how the calculation of the total area is performed, the area of the thread going through a cell, and the uncovered area for each of the cells.

<u>Detailed Description of Preferred Embodiments</u>

The present invention provides a method for calculating the final position of interlaced devices with one or more singularities. The selection of an appropriate endosaccular device size is crucial for a successful treatment and strongly depends of the final configuration that the device adopts when it adapts to the aneurysm sac morphology. This is frequently a problem during the intervention, leading to replacement of the device, reopening of the aneurysm or a need for re-treatment. A technique that allows predicting the released device configuration before intervention provides a powerful computational tool to aid the interventionist during device selection.

The method is based on the analysis of the local morphology of the region to be treated, more specifically on the analysis of how the interlaced device is deformed as it adapts to the local morphology of the vessel, for example an aneurysm. The morphological description of the aneurysm and the descriptive specifications of the interlaced device design (height, diameter, amount of threads, etc.) are also preferably taken into account by the proposed method.

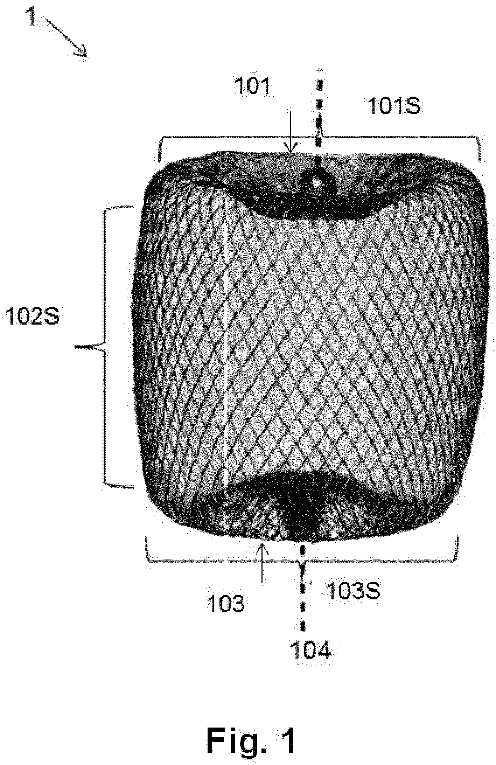

In reference to Fig. 1, said figure shows an example of an interlaced device 1 with three different sections, a proximal section 103S with a singularity (even though the threads are not observed), a central 102S tubular-shaped and a distal section 101S with a singularity (even though the threads are not observed).

The interlaced device 1 of Fig. 1 is similar to a WEB (Woven Endo Bridge) type device such as the one described in documents US 20120283768-A1 and US 10136896-B2. In this particular case, the interlaced device 1 is closed at both ends and the interlaced device is considered to be characterized by said three sections 101S, 102S, 103S.

It should be noted that the proposed method is generic and can be simplified to as many sections as desired; in fact, it is only necessary for one of the ends 101, 103 of the interlaced device 1 to include a singularity. The interlaced device 1 can also be attached to a second interlaced device through the proximal end 103 or distal end 101.

In this case, it is only necessary for contiguous sections to coincide with one another in type (singularity or open) and in size (the same diameters). The interlacing configuration and design that the threads of the interlaced device 1 have in each of its sections, particularly in the proximal and distal sections 103S, 101S, can thereby especially be considered.

In the interlaced device 1, the threads in the sections 101S, 103S with singularities extend radially, all of said threads being attached to a point referred to as the hub, located on axis 104 of the interlaced device 1. The threads in these sections 101S, 103S are constituted by adopting a sinusoidal shape in the radial direction when the interlaced device is deployed outside the catheter. The position of the threads in the radial direction can be exemplified using a sinusoidal function for the interlaced device 1. Nevertheless, other functions such as a sinusoidal function with an exponential decay, a logarithmic function, a catenary function, an exponential function, etc., could also be used.

Local morphology of the vessel can be quantified using morphological descriptors that are computed semi-automatically using state-of-the-art software.

In this description, the term "nominal morphological descriptor $M_n$" is used to refer to the magnitude achieved by a descriptor of the morphology of the interlaced device 1, such as radius or height, when the device is released outside of a vascular structure or of the positioning device (catheter). This configuration of the interlaced device 1 is referred to as the nominal (free) configuration. Therefore, for example, if the radius is considered as a morphological descriptor of the interlaced device 1, the nominal radius is the radius it will adopt when it is completely free, coinciding with the maximum radius it may reach.

On the other hand, the morphological descriptors $m_d$ and $m_a$ (and also $m_c$ if this descriptor is calculated) can include any of: the minimum radius of the cross-section of the vasculature perpendicular to the central line, the maximum radius, the radius of the equivalent circumference with the perimeter equal to the cross-section perpendicular to the central line, the radius of the equivalent circumference with an area equal to the cross-section perpendicular to the central line, etc., or combinations of the foregoing.

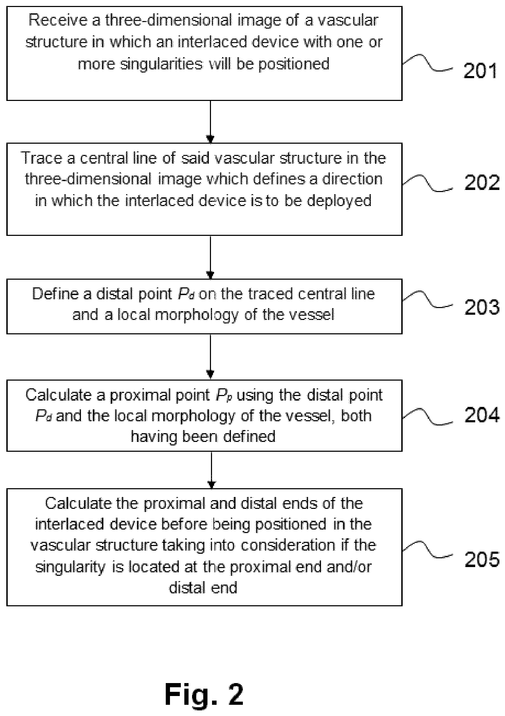

Fig. 2 illustrates an embodiment of a method for calculating the proximal and distal end of an interlaced device before being positioned in a vascular structure. The method in step 201 comprises receiving a three-dimensional image of a vascular structure in which a device formed by interlaced threads, or interlaced device, with one or more singularities (for example the interlaced device 1 of Fig. 1), will be positioned. In step 202, the method comprises tracing a central line of said vascular structure in the three-dimensional image which defines a direction in which the interlaced device 1 is to be deployed. This direction may or may not be the main direction of the vessel (or aneurysm), according to the configuration thereof and the manner of accessing same with the catheter. Then, in step 203, the method comprises defining a distal point $P_d$ on the traced central line and a local morphology of the vessel. The distal point $P_d$ indicates the point where the distal end 101 of the interlaced device 1 will start to be deployed. In step 204, a proximal point $P_p$ is calculated using the distal point $P_d$ and the local morphology of the vessel, both having been defined. The proximal point $P_p$ indicates the point that limits a portion of the traced central line over the traced central line that will be needed for deploying the mentioned section of the interlaced device including the singularity. Finally, in step 205, the proximal and distal ends of the interlaced device 1 are calculated taking into consideration whether the singularity is located at the proximal end 103 and/or distal end 101.

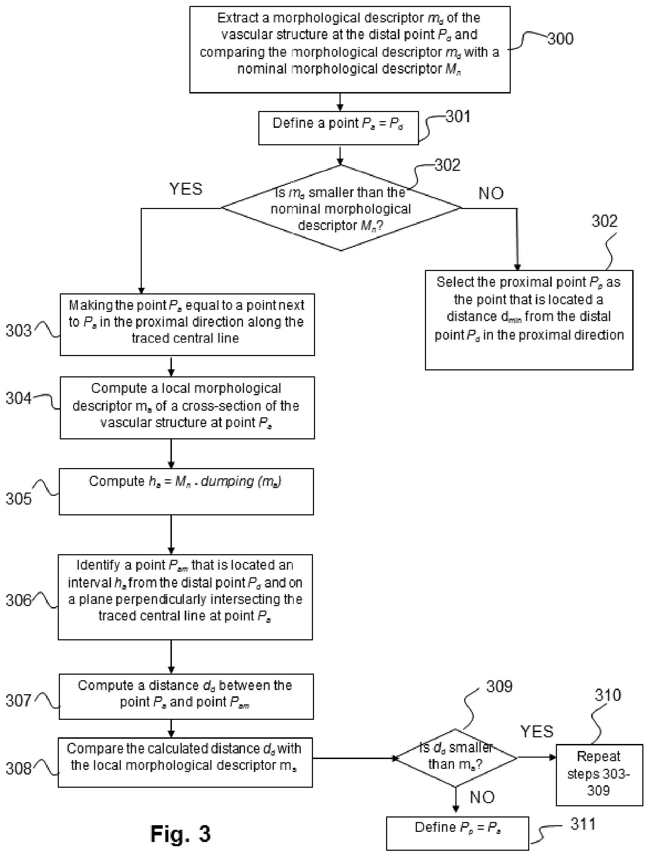

Fig. 3 illustrates an embodiment of the calculation of the singularity if the latter is located in the distal section of an interlaced device. The method comprises extracting (step 300) a morphological descriptor $m_d$ of the vascular structure at the distal point $P_d$ and comparing the morphological descriptor $m_d$ with a nominal morphological descriptor $M_n$ of the distal section 101S of the interlaced device 1. At step 301, a point $P_a$ is defined as $P_a = P_d$.

If the morphological descriptor $m_d$ is smaller than the nominal morphological descriptor $M_n$, the method further comprises (step 303) making the point $P_a$ equal to a point next to $P_a$ in the proximal direction along the traced central line, calculating (step 304) a local morphological descriptor $m_a$ of a cross-section of the vascular structure at said point $P_a$, and calculating (step 305) $h_a = M_n * dumping (m_a)$, where $h_a$ refers to a distance, and *dumping(m)* is a mathematical function in the interval [0,1] that accounts for the variation of $h_a$ when the interlaced device 1 has different expansion diameters (or simply expansions).

The relation of the device expansion and the height of the distal section 101S having the singularity is nonlinear. Thus, the damping function accounts for this nonlinearity by "damping" (i.e. multiplying by a number between 0 and 1) the nominal morphological descriptor $M_n$ to obtain the height $h_a$. In the case of a linear relation between the device expansion and its height, the damping will be constant for different values of $m_a$. It might also be equal to 1, depending on the design and behavior of the interlaced device 1. Consequently, the expansion diameter (or simply expansion) is the diameter achieved by the interlaced device 1 and can go from 0 (the interlaced device 1 is fully closed, e.g. inside the catheter) to the nominal diameter (i.e. the diameter of the interlaced device 1 when is fully opened, with the maximum diameter). In this particular embodiment, the expansion diameter is governed by $m_a$. The larger the vessel is the larger $m_a$ and the larger expansion will be, but not being larger than the nominal diameter.

In step 306, the method comprises identifying a point $P_{am}$ that is located an interval $h_a$ from the distal point $P_d$ and on a plane perpendicularly intersecting the traced central line at point $P_a$.

In step 307, a distance $d_d$ between the point $P_a$ and point $P_{am}$ is calculated, and the calculated distance $d_d$ is compared (step 308) with the local morphological descriptor $m_a$. If $d_d$ is smaller (step 310) than the local morphological descriptor $m_a$, steps 303-309 are repeated. Otherwise, (step 311) $P_p = P_a$ is defined as the proximal point that limits a portion of the central line over the traced central line that will be needed for deploying the distal section 101S of the interlaced device 1.

It should be noted that the point $P_a$ refers to the current point being studied at a given iteration of the algorithm, as a candidate to release the distal section 101S. The point $P_a$ is iteratively searched, along the traced central line, advancing one small step at a time in the proximal direction. Because $P_a$ candidates are predefined in some embodiments it can happen that a point $P_a$ satisfying $d_d = m_a$ might not exist. So, associated $P_{am}$ to $P_a$ should be such that the $d_d => h$, in the iterative search.

If the morphological descriptor $m_d$ is greater than or equal to the nominal morphological descriptor $M_n$, the method comprises (step 302) selecting the proximal point $P_p$ as the point that is located a distance $d_{min}$ from the distal point $P_d$ in the proximal direction, where $d_{min}$ is the minimum height, in the direction of the traced central line, achieved by said distal section 101S of the interlaced device 1, corresponding to the height of the section 101S being in the configuration corresponding to the nominal morphological descriptor $M_n$.

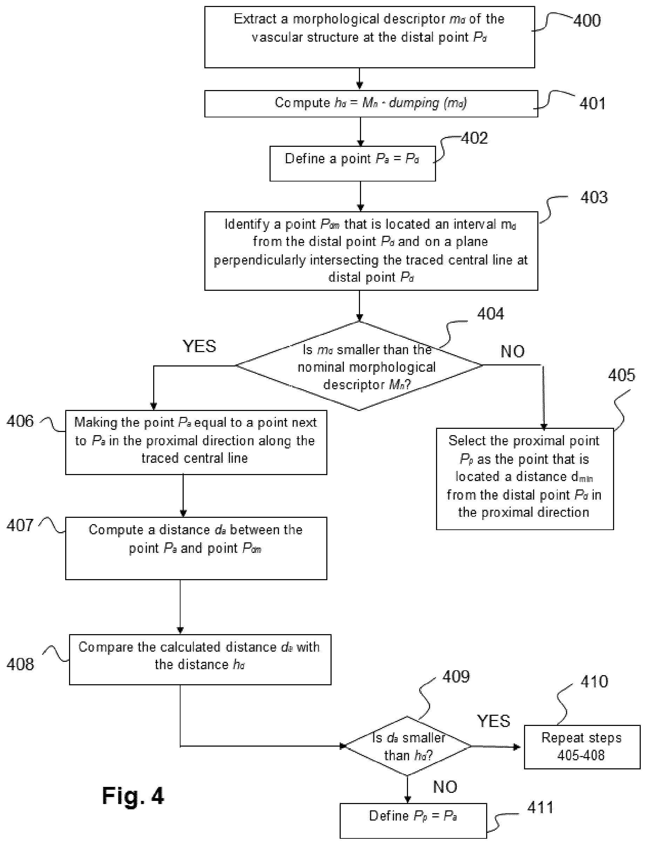

Fig. 4 illustrates an embodiment of the calculation of the singularity if the latter is in the proximal section of an interlaced device. In step 400, the method comprises extracting a morphological descriptor $m_d$ of the vascular structure at the distal point $P_d$. Then, in step 401, $h_d = M_n * dumping (m_d)$ is calculated, where $h_d$ is a distance, and *dumping(m)* is a mathematical function in the interval [0,1] which in this case considers the variation of $h_d$ according to the expansion of the proximal section 103S of the interlaced device 1 (i.e. the section having the singularity in this case), to an expansion diameter corresponding to the morphological descriptor $m_d$. Different to the embodiment of Fig. 3, in this case, the expansion diameter is governed by $m_d$.

In step 402, a point $P_a$ is defined as $P_a = P_d$. Then, in step 403, the method comprises identifying a point $P_{dm}$ that is located an interval $m_d$ from the distal point $P_d$ and on a plane perpendicularly intersecting the traced central line at the distal point $P_d$.

In step 404, the method comprises comparing the morphological descriptor $m_d$ with a nominal morphological descriptor $M_n$.

If the morphological descriptor $m_d$ is smaller than the nominal morphological descriptor $M_n$, the method comprises making (step 406) the point $P_a$ equal to a point next to $P_a$ in the proximal direction along the traced central line, calculating (step 407) a distance $d_a$ between point $P_a$ and point $P_{dm}$, and comparing (step 408) the calculated distance $d_a$ with the distance $h_d$.

If the distance $d_a$ is smaller than the distance $h_d$ (step 409), the preceding steps 406-408 are repeated (step 410). Otherwise, in step 411, $P_p = P_a$ is defined as the proximal point that limits a portion of the central line over the traced central line that will be needed for deploying the proximal section 103S of the interlaced device 1.

As outlined for the section with a singularity at the distal end 101, it should be noted that the point $P_a$ refers to the current point being studied at a given iteration of the algorithm, as a candidate to release the distal section 101S. The point $P_a$ is iteratively searched, along the traced central line, advancing one small step at a time in the proximal direction. Because $P_a$ candidates are predefined in some embodiments it can happen that the point $P_a$ satisfying that $d_a = h_d$ might not exist. So, associated $P_{dm}$ to $P_a$ should be such that $d_a => h_d$ in the iterative search.

If the morphological descriptor $m_d$ is greater than or equal to the nominal morphological descriptor $M_n$ (step 404), the method comprises selecting the proximal point $P_p$ as the point that is located a distance $d_{min}$ from the distal point $P_d$ in the proximal direction, where $d_{min}$ is the minimum height, over the traced central line, achieved by the proximal section 103S, corresponding to the height of said section 103S being in the configuration corresponding to the morphological descriptor $m_d$.

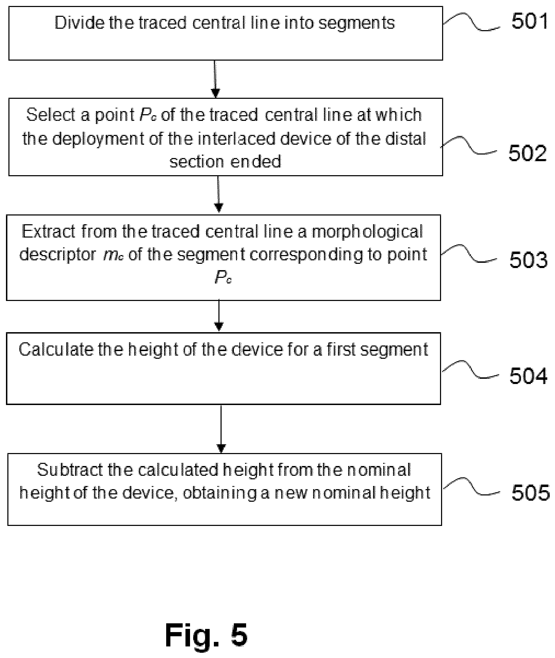

In the event that the interlaced device is identical to the device of Fig. 1 and includes a tubular-shaped central section, the methodology to be implemented according to one embodiment would be the one described in Fig. 5. First the traced central line of the vascular structure is divided into different segments (step 501). Then, in step 502, a point $P_c$ of the traced central line at which deployment of the interlaced device 1 of the distal section 101S ended is taken, and one or more morphological descriptors $m_c$ of the segment corresponding to point $P_c$ is/are extracted (step 503) from the traced central line. Next, in step 504, the height of the interlaced device 1 for a first segment is calculated, particularly using an indicator ratio. The indicator function determines a change in height of the interlaced device 1 according to the local morphology of the vascular structure. This morphological information is obtained from the description of the interlaced device design, for example, amount of threads, interlacing angle, length of the threads, height and diameter of the interlaced device deployed outside of the vessel, etc.). In step 505, the calculated height is subtracted from the nominal height of the interlaced device 1, obtaining a new nominal height that will be used in the following iteration of the method.

If the new nominal height is greater than 0, steps 503 to 505 are repeated for the segment contiguous to the preceding segment, moving forward in the proximal direction. If the new nominal height is approximately 0 (smaller than the separation between points of the traced central line), the lengths of all the segments in respect of which it moved forward are added together, this sum being the final height of the interlaced device 1 after its positioning.

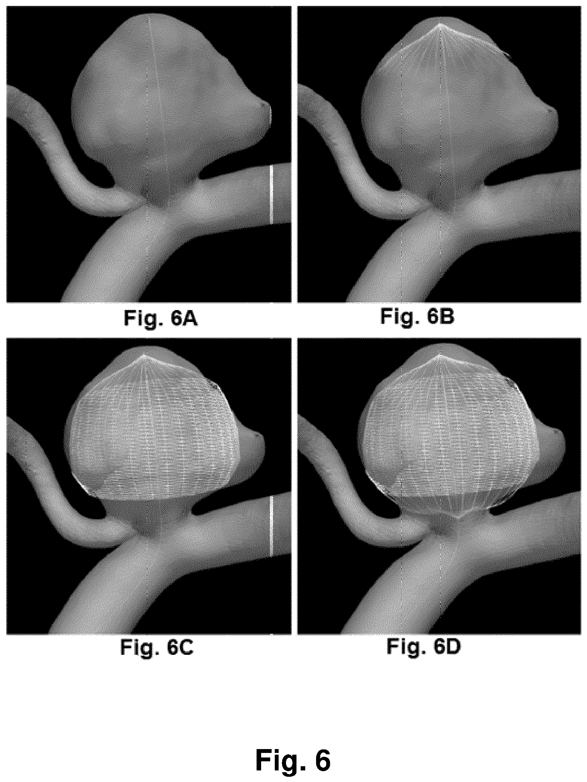

Fig. 6 illustrates the result obtained upon application of the proposed method. In Fig. 6D, the final arrangement of the interlaced device 1 implanted in the vascular structure can be observed.

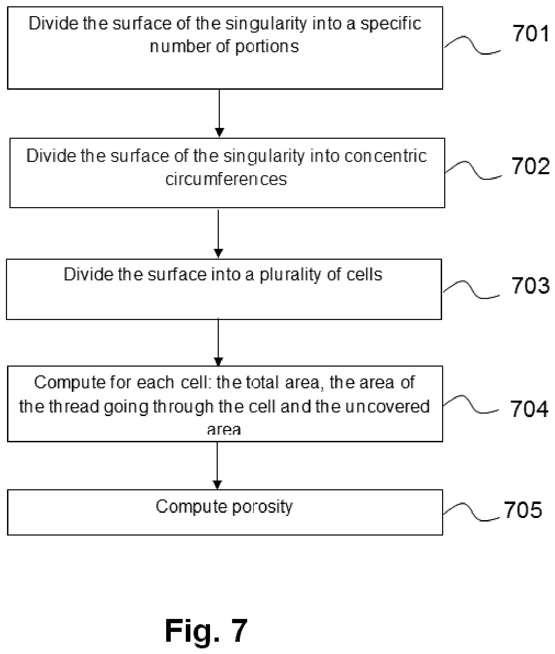

Fig. 7 shows an embodiment of the calculation of the porosity of the interlaced device 1, with one or more of its ends closed, not only on the sides thereof but also at the sections (singularities). In step 701, the method comprises dividing the surface of the singularity into a specific number of portions, or unitary portions 81. The number of portions 81 has a common center 80 at the distal point $P_d$, cover the entire mentioned surface, and depend on the number of interlaced threads constituting the interlaced device 1. In one embodiment, the surface of the singularity is $N_h$, where $N_h$ is the total number of threads constituting the device, obtaining the surface of the singularity partitioned into straight lines converging at the hub, equidistant at an angle $\Delta\theta = \dfrac{2\pi}{N_h}$.

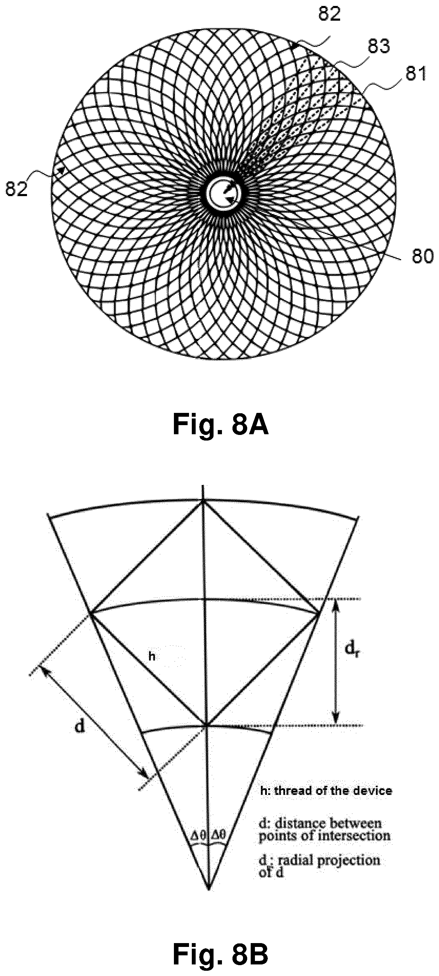

In step 702, the method comprises dividing the surface of the singularity into concentric circumferences 82, wherein the circumferences have a common center at the distal point $P_d$, and subsequently dividing (step 703) the surface into a plurality of cells 83. Each cell is obtained particularly considering the section of one of said portions 81 contained between two consecutive concentric circumferences 82. Fig. 8A graphically shows the preceding steps 701-703.

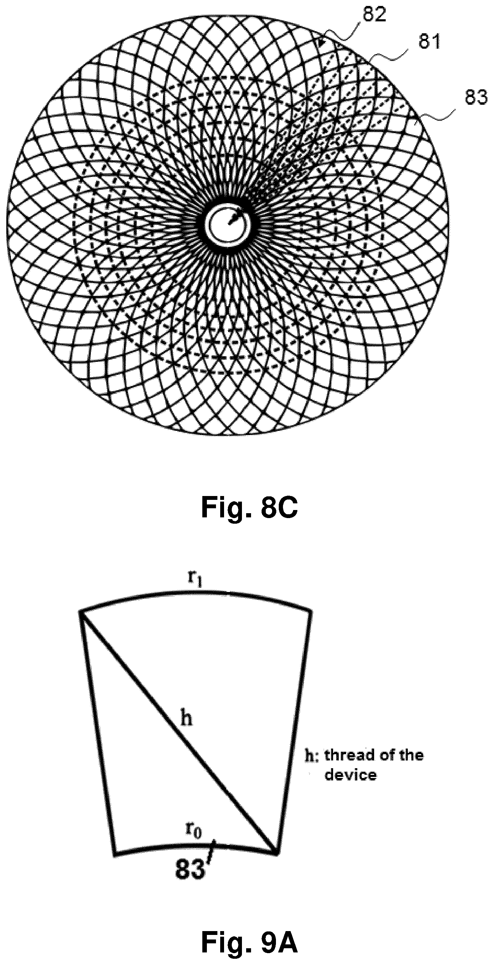

Fig. 8B illustrates the ratio of the radii of the circumference of Fig. 8A, the radii being equal to the radial projection $d_r$ of the distance d between points of intersection. Concentric rings partitioned by the straight lines converging at the hub considered in step 701 are thereby obtained (see Fig. 8C). Each of the partitions represents a cell 83. In one embodiment, the polar coordinate system is used with the pole being the hub of the interlaced device 1 and the polar axis being one of the converging straight lines.

Continuing with the methodology of Fig. 7, in step 704, the method comprises calculating for each cell 83: the total area, the area of the thread going through the cell 83, and the uncovered area for each of the cells 83.

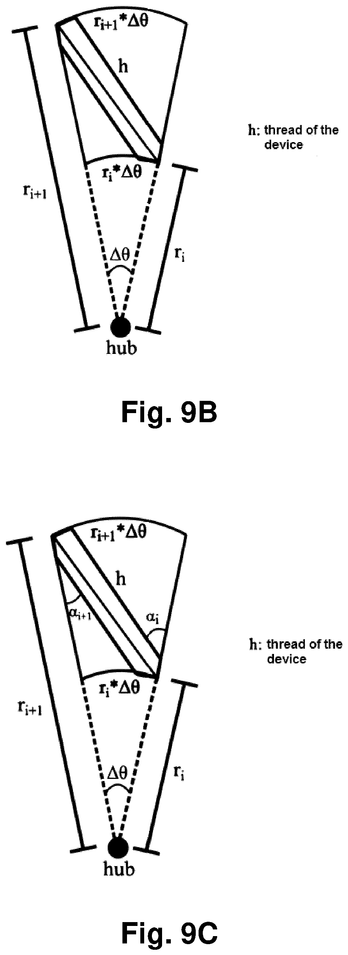

Figs. 9A-9C illustrate an example of performing the calculation of the total area, the area of the thread going through the cell 83, and the uncovered area for each of the cells 83. To that end, as shown in Fig. 9A, the circumference of radius $r_1 = d_{r_1}$ is considered. In this initial step, $r_0$ is the circumference of the hub of the interlaced device 1. In polar coordinates, the area of the cell 83 is calculated (see Fig. 9B):

$$A_c = \int_{\theta_1}^{\theta_2} \frac{1}{2}(r_1^2 - r_0^2)d\theta$$

For the partition performed, the variation $d\theta$ of the angle is constant $\Delta\theta$, then:

$$A_c = \frac{1}{2}(r_1^2 - r_0^2)\Delta\theta$$

In general for the cell $i$, i.e., for the circumference of radius $r_i = d_{r_i}$, the area is calculated:

$$A_c = \frac{1}{2}\left(r_i^2 - r_{i-1}^2\right)\Delta\theta$$

where $r_{i-1}$ is the radius of the circumference taken in step i - 1 and constituting the lower section of the current cell. In one embodiment, in each concentric circumference 82 the total number of cells 83 is $N_h$ and the area is the same in all of them. The area of the uncovered surface of the cell 83 depends on the angle $\alpha$ formed by the thread enclosed in cell 83 with the radial direction.

$$A_{uncovered} = A_c - A_h$$

where $A_c$ is the total area of the cell 83 and $A_h$ is the area of the thread inside the cell 83.

The area of the uncovered surface consists of two "triangles" the surfaces of which depend on angles $\alpha_i$ and $\alpha_{i-1}$ (see Fig. 9C).

Finally, in step 705, the porosity of each cell 83 is calculated as the ratio between the uncovered area divided by the total area of the cell 83 or the ratio between the covered area divided by the total area of the cell 83.

The proposed invention can be implemented in hardware, software, firmware, or any combination thereof. If it is implemented in software, the functions can be stored in or encoded as one or more code instructions in a computer-readable medium.

The scope of the present invention is defined in the attached claims.

## Claims

1. A computer-implemented method for calculating proximal and distal ends of an interlaced device before being positioned in a vascular structure, comprising:

   - receiving, by a computer, a three-dimensional image of a vascular structure in which a device formed by interlaced threads, also termed interlaced device (1), will be positioned, and tracing a central line of said vascular structure in the three-dimensional image which defines a direction in which the interlaced device (1) is to be deployed, the interlaced device (1) including at a proximal end (103) or at a distal end (101) thereof a section (103S, 101S) where a plurality of the interlaced threads coincide, also termed singularity;

**characterized in that** the method further comprises:

- defining, by the computer, based on an input provided by a user, a distal point $P_d$ on the traced central line and a local morphology of a vessel, the distal point $P_d$ indicating the point where the distal end (101) will start to be deployed and the local morphology of the vessel being the shape of the vessel in the vicinity of a given point;
- calculating, by the computer, a proximal point $P_p$ using the defined distal point $P_d$ and the defined local morphology of the vessel, wherein the proximal point $P_p$ indicates the point that limits a portion of the central line over the traced central line that will be needed for deploying said section (103S, 101S) of the interlaced device (1) including the singularity, wherein:

• if the singularity is at the distal end (101), the method further comprises extracting a morphological descriptor $m_d$ of the vascular structure at the distal point $P_d$ and comparing said morphological descriptor $m_d$ with a nominal morphological descriptor $M_n$ of a distal section (101S) of said sections (103S, 101S) of the interlaced device (1) including the singularity, the nominal morphological descriptor referring to a magnitude achieved by a descriptor of the morphology of the interlaced device (1), including the radius or the height, when the interlaced device (1) is released outside of the vascular structure or of a positioning device, wherein:

◦ a point $P_a$ is defined as $P_a = P_{d'}$ ;
◦ if the morphological descriptor $m_d$ is smaller than the nominal morphological descriptor $M_n$, the method further comprises:

i. making the point $P_a$ equal to a point next to $P_a$ in a proximal direction along the traced central line,
ii. calculating a local morphological descriptor $m_a$ of a cross-section of the vascular structure at said point $P_a$,
iii. calculating a distance $h_a$ as $h_a = M_n * dumping (m_a)$,
where *dumping(m)* is a mathematical function in the interval [0,1] which considers the variation of $h_a$ according to the expansion of said distal section (101S) to an expansion diameter corresponding to the local morphological descriptor $m_a$, the expansion diameter being the diameter achieved by the interlaced device (1) and ranging from 0, with the interlaced device (1) being fully closed, to the nominal diameter, with the interlaced device (1) being fully opened,
iv. identifying a point $P_{am}$ that is located an interval $h_a$ from the distal point $P_d$ and on a plane perpendicularly intersecting the traced central line at point $P_a$,
v. calculating $d_d$ as the distance between the point $P_a$ and the point $P_{am}$, and
vi. comparing the calculated distance $d_d$ with the local morphological descriptor $m_a$, wherein:

if the distance $d_d$ is smaller than the local morphological descriptor $m_a$, steps i. to v. are repeated,
if the distance $d_d$ is greater than or equal to the local morphological descriptor $m_a$, $P_p = P_a$ is defined as the proximal point that limits a portion of the central line over the traced central line that will be needed for deploying the distal section (101S); or

o if the morphological descriptor $m_d$ is greater than or equal to the nominal morphological descriptor $M_n$, the method comprises selecting the proximal point $P_p$ as the point that is located a distance $d_{min}$ from the distal point $P_d$ in the proximal direction, where $d_{min}$ is the minimum height, over the traced central line, achieved by said distal section (101S), corresponding to the height of the distal section (101S) being in the configuration corresponding to the nominal morphological descriptor $M_n$, which is the configuration where the interlaced device (1) is open to its maximum diameter, without constraints; or

• if the singularity is at the proximal end (103), the method further comprises:

- extracting a morphological descriptor $m_d$ of the vascular structure at the distal point $P_d$;
- calculating a distance $h_d$ as $h_d = M_n * dumping (m_d)$,

where *dumping(m)* is a mathematical function in the interval [0,1] which considers the variation of $h_d$

according to the expansion of a proximal section (103S) of said sections (103S, 101S) of the interlaced device (1) including the singularity to an expansion diameter corresponding to the morphological descriptor $m_d$; and

comparing said morphological descriptor $m_d$ with a nominal morphological descriptor $M_n$ of the proximal section (103S), wherein:

○ a point $P_a$ is defined as $P_a = P_d$;

o identifying a point $P_{dm}$ that is located an interval $m_d$ from the distal point $P_d$ and on a plane perpendicularly intersecting the traced central line at the distal point $P_d$,

○ if the morphological descriptor $m_d$ is smaller than the nominal morphological descriptor $M_n$, the method further comprises:

vii. making the point $P_a$ equal to a point next to $P_a$ in a proximal direction along the traced central line,

viii. calculating $d_a$ as the distance between the point $P_a$ and the point $P_{dm}$, and

iv. comparing the calculated distance $d_a$ with the distance $h_d$, wherein:

if the distance $d_a$ is smaller than the distance $h_d$, steps vii. to viii. are repeated,

if the distance $d_a$ is greater than or equal to the distance $h_d$, $P_p = P_a$ is defined as the proximal point that limits a portion of the central line over the traced central line that will be needed for deploying the proximal section (103S); or

o if the morphological descriptor $m_d$ is greater than or equal to the nominal morphological descriptor $M_n$, the method comprises selecting the proximal point $P_p$ as the point that is located a distance $d_{min}$ from the distal point $P_d$ in the proximal direction, where $d_{min}$ is the minimum height, over the traced central line, achieved by said proximal section (103S), corresponding to the height of said proximal section (103S) being in the configuration corresponding to the morphological descriptor $m_d$,

so that the final arrangement of the interlaced device (1) is known before the interlaced device (1) is implanted in the vascular structure.

2. The method according to claim 1, wherein the interlaced device (1) includes a singularity in the proximal section (103S) and a singularity in the distal section (101S), and wherein the calculation of the proximal and distal ends of the interlaced device (1) is performed for both proximal and distal sections (103S, 101S), being the distal point $P_d$ of the proximal section (103S) defined as the proximal point $P_p$ of the distal section (101S).

3. The method according to claim 2, wherein the interlaced device (1) further includes a central section (102S) thereof, and wherein the traced central line of the vascular structure is divided into different segments, wherein the method further comprises:

x. selecting a point $P_c$ of the traced central line at which deployment of the interlaced device (1) of the distal section (101S) ended, wherein the point $P_c$ is the proximal point $P_p$ of the distal section (101S);

xi. extracting from the traced central line at least one morphological descriptor $m_c$ of the segment corresponding to point $P_c$;

xii. calculating a height of the interlaced device (1) for a first segment using a ratio indicating a change in height of the interlaced device (1) according to the local morphology of the vascular structure;

xiii. subtracting said calculated height from a nominal height of the interlaced device (1), obtaining a new nominal height,

wherein if said new nominal height is greater than 0, steps xi. to xiii. are repeated for the segment contiguous to the preceding segment, moving forward in the proximal direction, and if the new nominal height is approximately 0, all the lengths of each segment are added together, this sum being the final height of the interlaced device (1) after its positioning.

4. The method according to any one of claims 1 to 3, further comprising:

attaching the interlaced device (1) to a second interlaced device at either the proximal end (103) or distal end (101) thereof, wherein the two interlaced devices are of the same size, and wherein the second interlaced device includes a singularity in its proximal section and/or in its distal section; and

performing the calculation of proximal and distal ends of the second interlaced device for at least one of said sections of the second interlaced device.

5. The method according to any one of the preceding claims, wherein the morphological descriptor $m_d$ comprises: the minimum radius of the cross-section of the vasculature perpendicular to the central line, or the maximum radius, or the radius of the equivalent circumference with the perimeter equal to the cross-section perpendicular to the central line, or the radius of the equivalent circumference with an area equal to the cross-section perpendicular to the central line.

6. The method according to any one of the preceding claims, wherein the local morphological descriptor $m_a$ comprises: the minimum radius of the cross-section of the vasculature perpendicular to the central line, or the maximum radius, or the radius of the equivalent circumference with the perimeter equal to the cross-section perpendicular to the central line, or the radius of the equivalent circumference with an area equal to the cross-section perpendicular to the central line.

7. The method according to any one of the preceding claims, further comprising:

dividing a surface of the singularity into a specific number of portions (81), wherein said number of portions (81) have a common center (80) at the distal point $P_d$, cover the entirety of said surface, and depend on the number of interlaced threads constituting the interlaced device (1);

dividing the surface into concentric circumferences (82), wherein said circumferences (82) have a common center at the distal point $P_d$;

dividing the surface into a plurality of cells (83), wherein each cell (83) is obtained considering a section of one of said portions (81) contained between two consecutive concentric circumferences (82);

calculating for each of the plurality of cells (83): a total area, an area of the thread going through the cell (83), and an uncovered area; and

calculating the porosity of each cell (83) as:

the ratio between the uncovered area divided by the total area of the cell (83); or
the ratio between the covered area divided by the total area of the cell (83).

8. The method according to claim 7, wherein all the portions (81) have an equal size.

9. The method according to claim 7, wherein all the portions (81) have a different size.

10. The method according to any one of claims 7 to 9, wherein the number of portions (81) is equal to the number of threads.

11. A non-transitory computer program product including code instructions which, when implemented in a processor of a computing device, implement a method according to any one of claims 1 to 10.


**Patentansprüche**

1. Computerimplementiertes Verfahren zum Berechnen des proximalen und distalen Endes einer verflochtenen Vorrichtung vor dem Positionieren in einer Gefäßstruktur, umfassend:

- Empfangen, durch einen Computer, eines dreidimensionalen Bildes einer Gefäßstruktur, in der eine aus verflochtenen Fäden gebildete Vorrichtung, auch verflochtene Vorrichtung (1) genannt, zu positionieren ist, und Zeichnen einer zentralen Linie der Gefäßstruktur in dem dreidimensionalen Bild, die eine Richtung definiert, in der die verflochtene Vorrichtung (1) eingesetzt werden soll, wobei die verflochtene Vorrichtung (1) an einem proximalen Ende (103) oder an einem distalen Ende (101) davon einen Abschnitt (103S, 101S) beinhaltet, in dem eine Vielzahl der verflochtenen Fäden zusammenfällt, auch als Singularität bezeichnet;

**dadurch gekennzeichnet, dass** das Verfahren ferner Folgendes umfasst:

- Definieren, durch den Computer, basierend auf einer Eingabe, die von einem Benutzer bereitgestellt wird, eines distalen Punktes $P_d$ auf der gezeichneten zentralen Linie und einer lokalen Morphologie eines Gefäßes, wobei der distale Punkt $P_d$ den Punkt anzeigt, an dem das distale Ende (101) beginnt, eingesetzt zu werden, und die lokale Morphologie des Gefäßes die Form des Gefäßes in der Nähe eines gegebenen Punktes ist;

- Berechnen, durch den Computer, eines proximalen Punktes $P_p$ unter Verwendung des definierten distalen Punktes $P_d$ und der definierten lokalen Morphologie des Gefäßes, wobei der proximale Punkt $P_p$ den Punkt anzeigt, der einen Teil der zentralen Linie über der gezeichneten zentralen Linie begrenzt, der für das Einsetzen des Abschnitts (103S, 101S) der verflochtenen Vorrichtung (1), die die Singularität beinhaltet, benötigt wird, wobei:

• falls sich die Singularität am distalen Ende (101) befindet, das Verfahren ferner das Extrahieren eines morphologischen Deskriptors $m_d$ der Gefäßstruktur an dem distalen Punkt $P_d$ und das Vergleichen des morphologischen Deskriptors $m_d$ mit einem nominalen morphologischen Deskriptor $M_n$ eines distalen Abschnitts (101S) der Abschnitte (103S, 101S) der verflochtenen Vorrichtung (1), die die Singularität beinhaltet, umfasst, wobei sich der nominale morphologische Deskriptor auf eine Größe bezieht, die durch einen Deskriptor der Morphologie der verflochtenen Vorrichtung (1), der den Radius oder die Höhe beinhaltet, erreicht wird, wenn die verflochtene Vorrichtung (1) außerhalb der Gefäßstruktur oder einer Positionierungsvorrichtung freigegeben wird, wobei:

o **ein Punkt** $P_a$ als $P_a = P_d$, definiert ist;
o falls der morphologische Deskriptor $m_d$ kleiner ist als der nominale morphologische Deskriptor $M_n$, das Verfahren ferner Folgendes umfasst:

i. Gleichsetzten des Punktes $P_a$ mit einem Punkt in der Nähe von $P_a$ in proximaler Richtung entlang der gezeichneten zentralen Linie,
ii. Berechnen eines lokalen morphologischen Deskriptors $m_a$ eines Querschnitts der Gefäßstruktur an diesem Punkt $P_a$,
iii. Berechnen einer Entfernung $h_a$ als $h_a = M_n \cdot dumping\,(m_a)$,
wobei $dumping(m)$ eine mathematische Funktion im Intervall $[0,1]$ ist, die die Variation von $h_a$ gemäß der Expansion des distalen Abschnitts (101S) auf einen Expansionsdurchmesser berücksichtigt, der dem lokalen morphologischen Deskriptor $m_a$ entspricht, wobei der Expansionsdurchmesser der Durchmesser ist, der durch die verflochtene Vorrichtung (1) erreicht wird und im Bereich von 0, wenn die verflochtene Vorrichtung (1) vollständig geschlossen ist, bis zum nominalen Durchmesser liegt, wenn die verflochtene Vorrichtung (1) vollständig geöffnet ist,
iv. Identifizieren eines Punktes $P_{am}$, der sich in einem Intervall $h_a$ vom distalen Punkt $P_d$ befindet und auf einer Ebene liegt, die die gezeichnete zentrale Linie im Punkt $P_a$ senkrecht schneidet,
v. Berechnen von $d_d$ als die Entfernung zwischen dem Punkt $P_a$ und dem Punkt $P_{am}$, und
vi. Vergleichen der berechneten Entfernung $d_d$ mit dem lokalen morphologischen Deskriptor $m_a$, wobei:

falls die Entfernung $d_d$ kleiner ist als der lokale morphologische Deskriptor $m_a$, die Schritte i. bis v. wiederholt werden,
falls die Entfernung $d_d$ größer oder gleich dem lokalen morphologischen Deskriptor $m_a$ ist, wird $P_p = P_a$ als der proximale Punkt definiert, der einen Teil der zentralen Linie über der gezeichneten zentralen Linie begrenzt, der für das Einsetzen des distalen Abschnitts (101S) benötigt wird; oder

o falls der morphologische Deskriptor $m_d$ größer oder gleich dem nominalen morphologischen Deskriptor $M_n$ ist, das Verfahren das Auswählen des proximalen Punktes $P_p$ als den Punkt umfasst, der sich in einer Entfernung $d_{min}$ vom distalen Punkt $P_d$ in der proximalen Richtung befindet, wobei $d_{min}$ die minimale Höhe ist, über der gezeichneten zentralen Linie, die durch den distalen Abschnitt (101S) erreicht wird, entsprechend der Höhe des distalen Abschnitts (101S), der sich in der Konfiguration befindet, die dem nominalen morphologischen Deskriptor $M_n$ entspricht, was die Konfiguration ist, in der die verflochtene Vorrichtung (1) bis zu ihrem maximalen Durchmesser offen ist, ohne Beschränkungen; oder

• falls sich die Singularität am proximalen Ende (103) befindet, das Verfahren ferner Folgendes umfasst:

- Extrahieren eines morphologischen Deskriptors $m_d$ der Gefäßstruktur am distalen Punkt $P_d$,

- Berechnen einer Entfernung $h_d$ als $h_d = M_n \cdot dumping\ (m_d)$,

wobei *dumping(m)* eine mathematische Funktion im Intervall [0,1] ist, die die Variation von $h_d$ gemäß der Expansion eines proximalen Abschnitts (103S) der Abschnitte (103S, 101S) der verflochtenen Vorrichtung (1), die die Singularität beinhaltet, auf einen Expansionsdurchmesser berücksichtigt, der dem morphologischen Deskriptor $m_d$ entspricht; und

Vergleichen des morphologischen Deskriptors $m_d$ mit einem nominalen morphologischen Deskriptor $M_n$ des proximalen Abschnitts (103S), wobei:

o ein Punkt $P_a$ als $P_a = P_d$ definiert ist;
o Identifizieren eines Punktes $P_{dm}$, der sich in einem Intervall $m_d$ vom distalen Punkt $P_d$ befindet und auf einer Ebene liegt, die die gezeichnete zentrale Linie im distalen Punkt $P_d$ senkrecht schneidet,
o falls der morphologische Deskriptor $m_d$ kleiner ist als der nominale morphologische Deskriptor $M_n$, das Verfahren ferner Folgendes umfasst:

vii. Gleichsetzten des Punktes $P_a$ mit einem Punkt in der Nähe von $P_a$ in proximaler Richtung entlang der gezeichneten zentralen Linie,
viii. Berechnen von $d_a$ als die Entfernung zwischen dem Punkt $P_a$ und dem Punkt $P_{dm}$, und
iv. Vergleichen der berechneten Entfernung $d_a$ mit der Entfernung $h_d$, wobei:

falls die Entfernung $d_a$ kleiner ist als die Entfernung $h_d$, die Schritte vii. bis viii. wiederholt werden,
falls die Entfernung $d_a$ größer oder gleich der Entfernung $h_d$ ist, wird $P_p = P_a$ als der proximale Punkt definiert, der einen Teil der zentralen Linie über der gezeichneten zentralen Linie begrenzt, der für das Einsetzen des proximalen Abschnitts (103S) benötigt wird; oder

o falls der morphologische Deskriptor $m_d$ größer oder gleich dem nominalen morphologischen Deskriptor $M_n$ ist, das Verfahren das Auswählen des proximalen Punktes $P_p$ als den Punkt umfasst, der sich in einer Entfernung $d_{min}$ vom distalen Punkt $P_d$ in der proximalen Richtung befindet, wobei $d_{min}$ die minimale Höhe ist, über der gezeichneten zentralen Linie, die durch den proximalen Abschnitt (103S) erreicht wird, entsprechend der Höhe des proximalen Abschnitts (103S), der sich in der Konfiguration befindet, die dem morphologischen Deskriptor $m_d$ entspricht,

so dass die endgültige Anordnung der verflochtenen Vorrichtung (1) bekannt ist, bevor die verflochtene Vorrichtung (1) in die Gefäßstruktur implantiert wird.

2. Verfahren nach Anspruch 1, wobei die verflochtene Vorrichtung (1) eine Singularität im proximalen Abschnitt (103S) und eine Singularität im distalen Abschnitt (101S) beinhaltet, und wobei das Berechnen des proximalen und distalen Endes der verflochtenen Vorrichtung (1) sowohl für den proximalen als auch den distalen Abschnitt (103S, 101S) durchgeführt wird, wobei der distale Punkt $P_d$ des proximalen Abschnitts (103S) als der proximale Punkt $P_p$ des distalen Abschnitts (101S) definiert ist.

3. Verfahren nach Anspruch 2, wobei die verflochtene Vorrichtung (1) ferner einen zentralen Abschnitt (102S) davon beinhaltet, und wobei die gezeichnete zentrale Linie der Gefäßstruktur in unterschiedliche Segmente unterteilt ist, wobei das Verfahren ferner Folgendes umfasst:

x. Auswählen eines Punktes $P_c$ der gezeichneten zentralen Linie, an dem der Einsatz der verflochtenen Vorrichtung (1) des distalen Abschnitts (101S) endete, wobei der Punkt $P_c$ der proximale Punkt $P_p$ des distalen Abschnitts (101S) ist;
xi. Extrahieren aus der gezeichneten zentralen Linie mindestens eines morphologischen Deskriptors $m_c$ für das Segment, das dem Punkt $P_c$ entspricht;
xii. Berechnen einer Höhe der verflochtenen Vorrichtung (1) für ein erstes Segment unter Verwendung eines Verhältnisses, das eine Änderung der Höhe der verflochtenen Vorrichtung (1) gemäß der lokalen Morphologie der Gefäßstruktur anzeigt;
xiii. Subtrahieren der berechneten Höhe von einer nominalen Höhe der verflochtenen Vorrichtung (1), wobei eine neue nominale Höhe erhalten wird,

wobei, falls die neue nominale Höhe größer als 0 ist, die Schritte xi. bis xiii. für das an das vorhergehende Segment angrenzende Segment wiederholt werden, wobei die Bewegung in proximaler Richtung vorwärts erfolgt, und, falls die neue nominale Höhe ungefähr 0 ist, alle Längen jedes Segments zusammen addiert werden, wobei diese Summe die endgültige Höhe der verflochtenen Vorrichtung (1) nach ihrer Positionierung ist.

4. Verfahren nach einem der Ansprüche 1 bis 3, ferner umfassend:

   Anbringen der verflochtenen Vorrichtung (1) an einer zweiten verflochtenen Vorrichtung entweder an dem proximalen Ende (103) oder dem distalen Ende (101) davon, wobei die beiden verflochtenen Vorrichtungen die gleiche Größe haben und wobei die zweite verflochtene Vorrichtung eine Singularität in ihrem proximalen Abschnitt und/oder in ihrem distalen Abschnitt beinhaltet; und
   Durchführen der Berechnung des proximalen und distalen Endes der zweiten verflochtenen Vorrichtung für mindestens einen der Abschnitte der zweiten verflochtenen Vorrichtung.

5. Verfahren nach einem der vorhergehenden Ansprüche, wobei der morphologische Deskriptor $m_d$ Folgendes umfasst: den minimalen Radius des Querschnitts des Gefäßsystems senkrecht zur zentralen Linie oder den maximalen Radius oder den Radius des äquivalenten Umfangs mit dem Perimeter gleich dem Querschnitt senkrecht zur zentralen Linie oder den Radius des äquivalenten Umfangs mit einer Fläche gleich dem Querschnitt senkrecht zur zentralen Linie.

6. Verfahren nach einem der vorhergehenden Ansprüche, wobei der lokale morphologische Deskriptor $m_a$ Folgendes umfasst: den minimalen Radius des Querschnitts des Gefäßsystems senkrecht zur zentralen Linie oder den maximalen Radius oder den Radius des äquivalenten Umfangs mit dem Perimeter gleich dem Querschnitt senkrecht zur zentralen Linie oder den Radius des äquivalenten Umfangs mit einer Fläche gleich dem Querschnitt senkrecht zur zentralen Linie.

7. Verfahren nach einem der vorhergehenden Ansprüche, ferner umfassend:

   Unterteilen einer Oberfläche der Singularität in eine bestimmte Anzahl von Teilen (81), wobei die Anzahl der Teile (81) einen gemeinsamen Mittelpunkt (80) am distalen Punkt $P_d$ aufweisen, die Gesamtheit der Oberfläche abdecken und von der Anzahl der verflochtenen Fäden abhängen, die die verflochtene Vorrichtung (1) ausmachen;
   Unterteilen der Oberfläche in konzentrische Umfänge (82), wobei die Umfänge (82) einen gemeinsamen Mittelpunkt am distalen Punkt $P_d$ aufweisen;
   Unterteilen der Oberfläche in eine Vielzahl von Zellen (83), wobei jede Zelle (83) unter Berücksichtigung eines Abschnitts eines der Teile (81), der zwischen zwei aufeinanderfolgenden konzentrischen Umfängen (82) enthalten ist, erhalten wird;
   Berechnen für jede der Vielzahl von Zellen (83): einer Gesamtfläche, einer Fläche des Fadens, der durch die Zelle (83) verläuft, und einer nicht abgedeckten Fläche; und
   Berechnen der Porosität jeder Zelle (83) als:
   das Verhältnis zwischen der nicht abgedeckten Fläche geteilt durch die Gesamtfläche der Zelle (83); oder das Verhältnis zwischen der abgedeckten Fläche geteilt durch die Gesamtfläche der Zelle (83).

8. Verfahren nach Anspruch 7, wobei alle Teile (81) die gleiche Größe aufweisen.

9. Verfahren nach Anspruch 7, wobei alle Teile (81) eine unterschiedliche Größe aufweisen.

10. Verfahren nach einem der Ansprüche 7 bis 9, wobei die Anzahl der Teile (81) gleich der Anzahl der Fäden ist.

11. Nicht-transitorisches Computerprogrammprodukt, das Codeanweisungen beinhaltet, die, wenn sie in einem Prozessor einer Rechenvorrichtung implementiert werden, ein Verfahren nach einem der Ansprüche 1 bis 10 implementieren.


**Revendications**

1. Procédé mis en œuvre par ordinateur pour calculer les extrémités proximale et distale d'un dispositif entrelacé avant son positionnement dans une structure vasculaire, comprenant :

- la réception, par un ordinateur, d'une image tridimensionnelle d'une structure vasculaire dans laquelle sera positionné un dispositif formé de fils entrelacés, également appelé dispositif entrelacé (1), et le traçage d'une ligne centrale de ladite structure vasculaire dans l'image tridimensionnelle, laquelle définit une direction dans laquelle le dispositif entrelacé (1) doit être déployé, le dispositif entrelacé (1) comprenant, à une extrémité proximale (103) ou à une extrémité distale (101), une section (103S, 101S) où une pluralité des fils entrelacés coïncident, également appelée singularité ;

**caractérisé en ce que** le procédé comprend en outre :

- la définition, par l'ordinateur, sur la base d'une donnée fournie par un utilisateur, d'un point distal $P_d$ sur la ligne centrale tracée et d'une morphologie locale d'un vaisseau, le point distal $P_d$ indiquant le point où l'extrémité distale (101) commencera à être déployée et la morphologie locale du vaisseau étant la forme du vaisseau à proximité d'un point donné ;
- le calcul, par l'ordinateur, d'un point proximal $P_p$ à l'aide du point distal $P_d$ défini et de la morphologie locale du vaisseau définie, le point proximal $P_p$ indiquant le point qui limite une portion de la ligne centrale sur la ligne centrale tracée, laquelle sera nécessaire pour le déploiement de ladite section (103S, 101S) du dispositif entrelacé (1) comprenant la singularité,
dans lequel :

- si la singularité se trouve à l'extrémité distale (101), le procédé comprend en outre l'extraction d'un descripteur morphologique $m_d$ de la structure vasculaire au point distal $P_d$ et la comparaison dudit descripteur morphologique $m_d$ avec un descripteur morphologique nominal $M_n$ d'une section distale (101S) desdites sections (103S, 101S) du dispositif entrelacé (1) comprenant la singularité, le descripteur morphologique nominal se rapportant à une grandeur obtenue par un descripteur de la morphologie du dispositif entrelacé (1), y compris le rayon ou la hauteur, lorsque le dispositif entrelacé (1) est libéré en dehors de la structure vasculaire ou d'un dispositif de positionnement, dans lequel :

  o un point $P_a$ est défini comme $P_a = P_d$ ;
  o si le descripteur morphologique $m_d$ est inférieur au descripteur morphologique nominal $M_n$, le procédé comprend en outre :

   i. faire en sorte que le point $P_a$ soit égal à un point voisin de $P_a$ dans une direction proximale le long de la ligne centrale tracée,
   ii. calculer un descripteur morphologique local $m_a$ d'une section transversale de la structure vasculaire audit point $P_a$,
   iii. calculer une distance $h_a$ telle que $h_a = M_a - dumping(m_a)$,
   où *dumping(m)* est une fonction mathématique dans l'intervalle [0,1] qui prend en compte la variation de $h_a$ en fonction de l'expansion de ladite section distale (101S) jusqu'à un diamètre d'expansion correspondant au descripteur morphologique local $m_a$, le diamètre d'expansion étant le diamètre atteint par le dispositif entrelacé (1) et variant de 0, lorsque le dispositif entrelacé (1) est totalement fermé, jusqu'au diamètre nominal, lorsque le dispositif entrelacé (1) est totalement ouvert,
   iv. identifier un point $P_{am}$ situé à un intervalle $h_a$ du point distal $P_d$ et sur un plan coupant perpendiculairement la ligne centrale tracée au point $P_a$,
   v. calculer $d_d$ comme la distance entre le point $P_a$ et le point $P_{am}$, et
   vi. comparer la distance dd calculée avec le descripteur morphologique local $m_a$, dans lequel :

    si la distance $d_d$ est inférieure au descripteur morphologique local $m_a$, les étapes i. à v. sont répétées,
    si la distance $d_d$ est supérieure ou égale au descripteur morphologique local $m_a$, $P_p = P_a$ est défini comme le point proximal qui limite une portion de la ligne centrale sur la ligne centrale tracée nécessaire au déploiement de la section distale (101S) ; ou

  o si le descripteur morphologique $m_d$ est supérieur ou égal au descripteur morphologique **nominal** $M_n$, le procédé comprend la sélection du point proximal $P_p$ comme étant le point situé à une distance $d_{min}$ du point distal $P_d$ dans la direction proximale, dmin étant la hauteur minimale, sur la ligne centrale tracée, atteinte par ladite section distale (101S), correspondant à la hauteur de la section distale (101S) dans la configuration correspondant au descripteur morphologique nominal $M_n$, laquelle est la configuration où le dispositif entrelacé (1) est ouvert à son diamètre maximal, sans contraintes ; ou

• si la singularité se trouve à l'extrémité proximale (103), le procédé comprend en outre :
l'extraction d'un descripteur morphologique $m_d$ de la structure vasculaire au point distal $P_d$,

- calculer une distance $h_d$ telle que $h_d = M_n{}^*$ dumping($m_d$),

où *dumping(m)* est une fonction mathématique dans l'intervalle [0,1] qui prend en compte la variation de $h_d$ en fonction de l'expansion d'une section proximale (103S) desdites sections (103S, 101S) du dispositif entrelacé (1) comprenant la singularité jusqu'à un diamètre d'expansion correspondant au descripteur morphologique $m_d$ ; et
la comparaison dudit descripteur morphologique $m_d$ avec un descripteur morphologique nominal $M_n$ de la section proximale (103S), dans lequel :

o un point $P_a$ est défini comme $P_a = P_d$ ;
o identification d'un point $P_{dm}$ situé à un intervalle $m_d$ du point distal $P_d$ et sur un plan coupant perpendiculairement la ligne centrale tracée au point distal $P_d$,
o si le descripteur morphologique $m_d$ est inférieur au descripteur morphologique nominal $M_n$, le procédé comprend en outre :

vii. faire en sorte que le point $P_a$ soit égal à un point voisin de $P_a$ dans une direction proximale le long de la ligne centrale tracée,
v. calculer $d_a$ comme la distance entre le point $P_a$ et le point $P_{dm}$, et
iv. la comparaison de la distance $d_a$ calculée avec la distance $h_d$, dans lequel :

si la distance $d_a$ est inférieure à la distance $h_d$, les étapes vii. à viii. sont répétées,
si la distance $d_a$ est supérieure ou égale à la distance $h_d$, $P_p = P_a$ est défini comme le point proximal qui limite une portion de la ligne centrale sur la ligne centrale tracée nécessaire au déploiement de la section proximale (103S) ; ou

o si le descripteur morphologique $m_d$ est supérieur ou égal au descripteur morphologique nominal $M_n$, le procédé comprend la sélection du point proximal $P_p$ comme étant le point situé à une distance $d_{min}$ du point distal $P_d$ dans la direction proximale, $d_{min}$ étant la hauteur minimale, sur la ligne centrale tracée, atteinte par ladite section proximale (103S), correspondant à la hauteur de ladite section proximale (103S) dans la configuration correspondant au descripteur morphologique $m_d$,

de telle sorte que l'arrangement final du dispositif entrelacé (1) soit connu avant que le dispositif entrelacé (1) ne soit implanté dans la structure vasculaire.

2. Procédé selon la revendication 1, dans lequel le dispositif entrelacé (1) comprend une singularité dans la section proximale (103S) et une singularité dans la section distale (101S), et dans lequel le calcul des extrémités proximale et distale du dispositif entrelacé (1) est effectué pour les sections proximale et distale (103S, 101S), le point distal $P_d$ de la section proximale (103S) étant défini comme le point proximal $P_p$ de la section distale (101S).

3. Procédé selon la revendication 2, dans lequel le dispositif entrelacé (1) comprend en outre une section centrale (102S), et dans lequel la ligne centrale tracée de la structure vasculaire est divisée en différents segments, le procédé comprenant en outre :

x. la sélection d'un point $P_c$ de la ligne centrale tracée au niveau duquel le déploiement du dispositif entrelacé (1) de la section distale (101S) a pris fin, le point $P_c$ étant le point proximal $P_p$ de la section distale (101S) ;
xi. l'extraction, à partir de la ligne centrale tracée, d'au moins un descripteur morphologique $m_c$ du segment correspondant au point $P_c$ ;
xii. le calcul d'une hauteur du dispositif entrelacé (1) pour un premier segment au moyen d'un rapport indiquant une variation de hauteur du dispositif entrelacé (1) en fonction de la morphologie locale de la structure vasculaire ;
xiii. la soustraction de ladite hauteur calculée à une hauteur nominale du dispositif entrelacé (1), obtenant une nouvelle hauteur nominale,

dans lequel, si ladite nouvelle hauteur nominale est supérieure à 0, les étapes xi. à xiii. sont répétées pour le segment contigu au segment précédent, en avançant dans la direction proximale, et si la nouvelle hauteur nominale est approximativement égale à 0, toutes les longueurs de chaque segment sont additionnées, cette somme constituant la hauteur finale du dispositif entrelacé (1) après son positionnement.

4.   Procédé selon l'une quelconque des revendications 1 à 3, comprenant en outre :

la fixation du dispositif entrelacé (1) à un second dispositif entrelacé, soit à son extrémité proximale (103), soit à son extrémité distale (101), les deux dispositifs entrelacés étant de la même taille, et le second dispositif entrelacé comprenant une singularité dans sa section proximale et/ou dans sa section distale ; et
l'exécution du calcul des extrémités proximale et distale du second dispositif entrelacé pour au moins une desdites sections du second dispositif entrelacé.

5.   Procédé selon l'une quelconque des revendications précédentes, dans lequel le descripteur morphologique $m_d$ comprend : le rayon minimal de la section transversale de la vascularisation perpendiculaire à la ligne centrale, ou le rayon maximal, ou le rayon de la circonférence équivalente dont le périmètre est égal à la section transversale perpendiculaire à la ligne centrale, ou le rayon de la circonférence équivalente dont la surface est égale à la section transversale perpendiculaire à la ligne centrale.

6.   Procédé selon l'une quelconque des revendications précédentes, dans lequel le descripteur morphologique $m_a$ comprend : le rayon minimal de la section transversale de la vascularisation perpendiculaire à la ligne centrale, ou le rayon maximal, ou le rayon de la circonférence équivalente dont le périmètre est égal à la section transversale perpendiculaire à la ligne centrale, ou le rayon de la circonférence équivalente dont la surface est égale à la section transversale perpendiculaire à la ligne centrale.

7.   Procédé selon l'une quelconque des revendications précédentes, comprenant en outre :

la division d'une surface de la singularité en un nombre spécifique de portions (81), dans lequel ledit nombre de portions (81) possède un centre commun (80) au point distal $P_d$, couvre l'intégralité de ladite surface et dépend du nombre de fils entrelacés constituant le dispositif entrelacé (1) ;
la division de la surface en circonférences concentriques (82), dans lequel lesdites circonférences (82) possèdent un centre commun au point distal $P_d$ ;
la division de la surface en une pluralité de cellules (83), dans lequel chaque cellule (83) est obtenue en considérant une section de l'une desdites portions (81) comprise entre deux circonférences concentriques (82) consécutives ;
le calcul, pour chacune de la pluralité de cellules (83) : d'une surface totale, d'une surface du fil traversant la cellule (83), et d'une surface découverte ; et
le calcul de la porosité de chaque cellule (83) comme :

le rapport entre la surface découverte divisée par la surface totale de la cellule (83) ;
ou le rapport entre la surface couverte divisée par la surface totale de la cellule (83).

8.   Procédé selon la revendication 7, dans lequel toutes les portions (81) ont une taille égale.

9.   Procédé selon la revendication 7, dans lequel toutes les portions (81) ont une taille différente.

10.  Procédé selon l'une quelconque des revendications 7 à 9, dans lequel le nombre de portions (81) est égal au nombre de fils.

11.  Produit-programme informatique non transitoire comprenant des instructions de code qui, lorsqu'elles sont exécutées dans un processeur d'un dispositif informatique, mettent en œuvre un procédé selon l'une quelconque des revendications 1 à 10.

**Fig. 1**

Receive a three-dimensional image of a vascular structure in which an interlaced device with one or more singularities will be positioned ⟶ 201

Trace a central line of said vascular structure in the three-dimensional image which defines a direction in which the interlaced device is to be deployed ⟶ 202

Define a distal point $P_d$ on the traced central line and a local morphology of the vessel ⟶ 203

Calculate a proximal point $P_p$ using the distal point $P_d$ and the local morphology of the vessel, both having been defined ⟶ 204

Calculate the proximal and distal ends of the interlaced device before being positioned in the vascular structure taking into consideration if the singularity is located at the proximal end and/or distal end ⟶ 205

# Fig. 2

Extract a morphological descriptor $m_d$ of the vascular structure at the distal point $P_d$ and comparing the morphological descriptor $m_d$ with a nominal morphological descriptor $M_n$ — 300

Define a point $P_a = P_d$ — 301

Is $m_a$ smaller than the nominal morphological descriptor $M_n$? — 302

YES

NO

302

Select the proximal point $P_p$ as the point that is located a distance $d_{min}$ from the distal point $P_d$ in the proximal direction

303 — Making the point $P_a$ equal to a point next to $P_a$ in the proximal direction along the traced central line

304 — Compute a local morphological descriptor $m_a$ of a cross-section of the vascular structure at point $P_a$

305 — Compute $h_a = M_n \cdot dumping\ (m_a)$

306 — Identify a point $P_{am}$ that is located an interval $h_a$ from the distal point $P_d$ and on a plane perpendicularly intersecting the traced central line at point $P_a$

307 — Compute a distance $d_a$ between the point $P_a$ and point $P_{am}$

308 — Compare the calculated distance $d_a$ with the local morphological descriptor $m_a$

309 — Is $d_a$ smaller than $m_a$?

YES

310 — Repeat steps 303–309

NO

Define $P_p = P_a$ — 311

**Fig. 3**

Extract a morphological descriptor $m_d$ of the
vascular structure at the distal point $P_d$ — 400

Compute $h_d = M_n \cdot dumping\ (m_d)$ — 401

Define a point $P_a = P_d$ — 402

Identify a point $P_{dm}$ that is located an interval $m_d$
from the distal point $P_d$ and on a plane
perpendicularly intersecting the traced central line at
distal point $P_d$ — 403

Is $m_d$ smaller than the
nominal morphological
descriptor $M_n$? — 404

YES

NO

405

Making the point $P_a$ equal to a point next
to $P_a$ in the proximal direction along the
traced central line — 406

Select the proximal point
$P_p$ as the point that is
located a distance $d_{min}$
from the distal point $P_d$ in
the proximal direction

Compute a distance $d_a$ between the
point $P_a$ and point $P_{dm}$ — 407

Compare the calculated distance $d_a$ with
the distance $h_d$ — 408

Is $d_a$ smaller
than $h_d$? — 409

YES — 410

Repeat steps
405-408

NO

Define $P_p = P_a$ — 411

**Fig. 4**

Divide the traced central line into segments — 501

Select a point $P_c$ of the traced central line at which the deployment of the interlaced device of the distal section ended — 502

Extract from the traced central line a morphological descriptor $m_c$ of the segment corresponding to point $P_c$ — 503

Calculate the height of the device for a first segment — 504

Subtract the calculated height from the nominal height of the device, obtaining a new nominal height — 505

# Fig. 5

Fig. 6A

Fig. 6B

Fig. 6C

Fig. 6D

# Fig. 6

```
┌─────────────────────────────────────────┐
│  Divide the surface of the singularity   │      701
│  into a specific number of portions      │
└─────────────────────────────────────────┘
                    │
                    ▼
┌─────────────────────────────────────────┐
│  Divide the surface of the singularity   │      702
│  into concentric circumferences          │
└─────────────────────────────────────────┘
                    │
                    ▼
┌─────────────────────────────────────────┐
│  Divide the surface into a plurality     │      703
│  of cells                                │
└─────────────────────────────────────────┘
                    │
                    ▼
┌─────────────────────────────────────────┐
│  Compute for each cell: the total area,  │      704
│  the area of the thread going through    │
│  the cell and the uncovered area         │
└─────────────────────────────────────────┘
                    │
                    ▼
┌─────────────────────────────────────────┐
│  Compute porosity                        │      705
└─────────────────────────────────────────┘
```

# Fig. 7

**Fig. 8A**

h: thread of the device

d: distance between points of intersection

d$_r$: radial projection of d

**Fig. 8B**

82
81
83

**Fig. 8C**

$r_1$

$h$

h: thread of the device

$r_0$

**83**

**Fig. 9A**

h: thread of the device

**Fig. 9B**

h: thread of the device

**Fig. 9C**

# EP 4 111 461 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120283768 A1
- US 10136896 B2
- US 10176566 B2
- US 2019038358 A1

**Non-patent literature cited in the description**

- **JR CEBRAL et al.** Analysis of flow dynamics and outcomes of cerebral aneurysms treated with intrasaccular flow-diverting devices. *American Journal of Neuroradiology*, 2019
- **FERNANDO MUT et al.** Image-based modeling of blood flow in cerebral aneurysms treated with intrasaccular flow diverting devices. *International journal for numerical methods in biomedical engineering*, 2019, e3202